# EUROPEAN PATENT SPECIFICATION

(11) **EP 3 668 388 B1**
(45) Date of publication and mention of the grant of the patent: **05.11.2025**
(21) Application number: 18846218.8
(22) Date of filing: 17.08.2018
(51) Int. Cl.: A61B 5/022, A61B 5/0225, A61B 5/023

(54) **HEART REFERENCE UNIT AND BLOOD PRESSURE MONITOR COMPRISING A HEART REFERENCE UNIT**
HERZREFERENZEINHEIT UND BLUTDRUCKMONITOR MIT EINER HERZREFERENZEINHEIT
UNITÉ DE RÉFÉRENCE CARDIAQUE ET DISPOSITIF DE SURVEILLANCE DE PRESSION ARTÉRIELLE COMPRENANT UNE UNITÉ DE RÉFÉRENCE CARDIAQUE

(30) Priority: 17.08.2017 US 201762546685 P; 14.08.2018 US 201816103579
(43) Date of publication of application: 24.06.2020
(73) Proprietor: Becton, Dickinson And Company, Franklin Lakes, New Jersey 07417 (US)
(72) Inventor: GUELEN, Ilja, Irvine, CA 92614 (US); VERSCHELLING, Marinus Cornelis, Jacobus, 3824 BC Amersfoort (NL)
(74) Representative: Eisenführ Speiser
(86) International application number: PCT/US2018/046867
(87) International publication number: WO 2019/036583

(56) References cited:
- WO-A1-2017/086945
- US-A- 4 779 626
- US-A- 5 103 832
- US-A- 5 769 083
- US-A- 5 957 853
- US-A1- 2006 036 184

## Description

The present invention relates to a blood pressure monitor for blood pressure measurement comprising a blood pressure sensor for measuring peripheral arterial pressure and a heart reference unit. The invention further relates to a heart reference unit for a blood pressure monitor according to invention.

Along with body temperature, respiratory rate, and pulse rate, blood pressure is one of the four main vital signs routinely monitored by medical professionals and healthcare providers. Blood pressure measurements are commonly performed at an extremity of the human body, such as an upper arm, a wrist or a finger. However, blood pressure readings are conventionally compared to reference central arterial blood pressure measured at heart level, as physicians are trained to derive treatment decisions from a blood pressure prevailing in the proximal arteries. This implies that when the location, and specifically the elevation, at which the blood pressure measurement is taken, is above or below heart level, an offset is obtained between the measured blood pressure and the central arterial blood pressure due to a hydrostatic pressure difference that exists between an artery at heart level and an artery at measurement level. Because of this offset, the blood pressure reading acquired cannot be compared directly to the reference central arterial blood pressure, as this would lead to inaccurate and/or incorrect results.

To adjust for the above-mentioned hydrostatic pressure difference, blood pressure meters exist that are equipped with a heart reference unit, also known as a height correction unit. Such a heart reference unit commonly comprises a fluid-filled tube that extends from the measurement location to the heart region or a heart level. A pressure sensor placed on an end of the tube then measures the hydrostatic pressure exerted by the fluid column within the fluid-filled tube. This pressure is taken as a measure for the difference in blood pressure that exists between the location where the blood pressure is obtained and the level of the heart. The blood pressure measured at a peripheral artery can herewith be adjusted accordingly. A drawback of this type of blood pressure meters is however that the equipped heart reference unit complicates the design of the blood pressure meter. Complications arise from the addition of the fluid-filled tube, as well as any electrical wires connecting the pressure sensor and other electrical components of the blood pressure meter. These complications not only render the manufacturing of said blood pressure meters more difficult, but also increase the chance of device malfunction. In addition, the added components, which need to be installed close to or routed along the patient, limit the patient's freedom of movement and therefore reduce the ergonomics of said blood pressure meters.

US 2006/036184 A1 describes a calibration system for pressure monitoring including a sensor positioned at a sensor location on or in a patient's body, a first pressure transducer positioned at a reference location remote from the sensor location to receive a signal from the sensor and to generate a first pressure signal, a calibration device positioned along a plane that is substantially coincident with a chamber or cavity (e.g., a heart chamber) of the patient to measure a reference pressure signal that represents a difference in pressure between the position of the calibration device and the reference location, a second pressure transducer positioned at the reference location remote from the sensor location to receive the reference pressure signal from the calibration device and to generate a calibration pressure signal, and an electronic device to produce an actual pressure signal using the first and calibration pressure signals.

US5957853A describes a biological pressure measuring system with a transmission device for transmitting a biological pressure at a biological location to a pressure monitor/ transducer for measuring the biological pressure, where an atmospheric pressure transmission device transmits an atmospheric pressure at a reference location to a reference pressure device for measuring atmospheric pressure and a vertical fluid pressure of the fluid in the atmospheric transmission device.

A goal of the present invention is therefore to provide a blood pressure monitor that can correct for hydrostatic pressure difference attributable to the difference in height of the blood pressure measurement location relative to the heart while at the same time being less cumbersome in use, easier to manufacture and/or more robust than known blood pressure monitors.

The object of the present invention is achieved with a stand-alone heart reference unit for a blood pressure monitor according to the independent claim. Various preferred embodiments of the invention are described in the dependent claims.

This disclosure also desribes a blood pressure monitor for non-invasive blood pressure measurement of the type stated in the preamble, comprising a heart reference unit comprising: a first fluid column, with a first end positionable at blood pressure sensor level, a second fluid column, with a first end positionable at a reference level, preferably the heart level, and at least one heart reference sensor for sensing the pressure prevailing in the first fluid column and the second fluid column, wherein a second end of the first fluid column and the second fluid column opposing the first ends of said fluid columns are positionable at a predetermined elevation relative to each other. As the blood pressure monitor comprises at least two separate fluid columns having only a first end to be positioned near the patient, the second ends of these fluid columns, which in a common instance are formed by fluid-filled tubes, may be routed to a suitable location away from the patient. These free second ends may then function as a connection location for the at least one heart reference sensor and any other components connectable to the fluid columns, at which location said components do not hinder the patient. At the same time, the operating principle of the heart reference unit remains similar to that of heart reference units incorporated in prior art blood pressure meters. Given that the second end of the first fluid column and the second fluid column opposing the first ends of said fluid columns have a known relative position, a difference in elevation between the blood pressure measurement location and the heart may be derived from the measured difference in hydrostatic pressure exerted by the fluid in the first and second fluid column. The blood pressure readings can accordingly be adjusted to represent a blood pressure in the proximal arteries. The blood pressure monitor may specifically be adapted for measuring peripheral arterial pressure at finger level, as space for the installation of additional components such as the heart reference sensor is limited. The blood pressure monitor is further eminently suited for monitoring continuous non-invasive arterial pressure as this typically requires measuring blood pressure over extended periods of time. The patient hereby especially benefits from the added convenience and freedom of movement the blood pressure monitor is able to offer over prior art alternatives, thereby increasing the comfort of the patient.

In an embodiment of the blood pressure monitor the heart reference unit comprises a differential pressure transducer for determining a hydrostatic pressure difference between the first fluid column and the second fluid column based on the pressures measured by the at least one heart reference sensor. This hydrostatic pressure difference is appropriately determined by subtracting the measured hydrostatic pressure exerted by the fluid in the first fluid column from the measured hydrostatic pressure exerted by the fluid in the second fluid column. The determined pressure difference is a measure for the difference in elevation between the first ends of the first and second fluid columns. Depending on the density of the fluid in the fluid columns and the relative positions of the second ends of the first and second fluid columns, the determined pressure difference could even directly reflect the pressure offset resulting from the column of blood present in the patient's artery running from the location of the blood pressure measurement to the heart. The differential pressure transducer could be implemented in the blood pressure monitor as a separate (electrical) component, but could also constitute part of the heart reference sensor, wherein the hydrostatic pressure difference forms the output of the heart reference sensor.

In a further embodiment, the blood pressure monitor further comprises a processor for deriving a central arterial pressure from the peripheral arterial pressure and the hydrostatic pressure difference. The central arterial pressure is obtained by subtracting the offset pressure, caused by the column of blood present in the patient's artery running from the location of the blood pressure measurement to the heart, from the peripheral arterial pressure as measured by the blood pressure sensor. The offset pressure either follows directly from the hydrostatic pressure difference between the pressures exerted by the fluid in the first and second fluid columns, which is the case if the second ends of the fluid columns are positioned at the same elevation and the density of the fluid contained in the fluid columns is the same as the density of the patient's blood, or may be indirectly determined from said hydrostatic pressure difference. In the latter case, the hydrostatic pressure difference must be corrected for known differences in fluid densities between the respective fluids in the first and second fluid columns and/or differences in densities between the fluids in the fluid columns and the patient's blood, as well as the difference in relative elevation of the second ends of the fluid columns. This correction may in this case as well be determined by the processor. The processor may also constitute part of the heart reference sensor. By integrating the processor into the blood pressure monitor, direct measurement processing becomes possible allowing for a real-time and local monitoring of a patient's blood pressure. The processor output could for this purpose be displayed on a suitable output device, such as a screen.

It is advantageous if the at least one heart reference sensor is positioned at the second ends of the fluid columns. When positioned at the second ends of the fluid columns, the at least one heart reference sensor could be positioned away from both the blood pressure sensor as well as the patient's body. Any wires connecting to the heart reference sensor can moreover be diverted away from the patient. This allows for a convenient connection and set-up process of the heart reference unit, with a minimal disturbance to the patient. In addition, the risk of the patient getting entangled in the (electrical) wiring connected to the heart reference sensor is herewith averted. Another advantage of positioning the at least one heart reference sensor at the second ends of the fluid this placement requires less manufacturing steps at the side of the blood pressure sensor. The blood pressure sensor could hereby retain a standard design, which has a positive effect on production costs and could benefit the interchangeability of parts.

The heart reference unit may comprise at least two heart reference sensors, respectively positioned at the second end of the first and the second fluid column, for respectively sensing the pressure prevailing in the first the second fluid column
By using two sensors to measure pressure to determine relative height, the system allows for an easier detection of leakages and improved replicability of parts, as the fluid columns can then be fully separated from each other. In a different embodiment, the at least one heart reference sensor may be a differential pressure sensor, and preferably a bidirectional differential pressure sensor, for sensing a difference in pressure prevailing in the first fluid column and the second fluid column. In the latter case, the second ends of the first fluid column and second fluid column may be mutually connected with the interposition of the differential pressure sensor. By using a differential pressure sensor, the heart reference unit can be limited to the use of a single heart reference sensor. The bidirectional sensor further allows both negative and positive differential pressures to be monitored in response to changes in hydrostatic pressure in the first and second fluid columns, whereby the hydrostatic pressure measured in the first fluid column may either be higher or lower than the hydrostatic pressure measured in the second fluid column.

In order to directly derive the difference in elevation between the first end of the first and second fluid columns from the measured hydrostatic pressure difference, the second ends of the first fluid column and the second fluid column may be positioned at a corresponding elevation. The difference in elevation between the first end of the first and second fluid columns is herewith reflected by the height of the fluid columns and therefore the hydrostatic pressure exerted by the fluid columns.

To ensure that the second ends of the fluid columns are retained at their predetermined relative (vertical) position, the second ends of the first fluid column and the second fluid column may be connected through a connector housing. The fluid columns may hereby be releasably connected to the connector housing, for example by using a click or snap connection, which connection is preferably secured against sudden disconnection. An added benefit of such connector housing is that it may contain (some of the) electronics and wiring for connecting the at least one heart reference sensor, which otherwise would be placed along the fluid columns. The connector housing may in addition house the at least one heart reference sensor. In this case, all electronic components may be contained within the housing, and no electronic connections are needed outside the connector housing. The connector housing in this instance thus significantly simplifies the construction of the heart reference unit, thereby simplifying the use of the blood pressure as well as facilitating its repair and maintenance.

In an advantageous embodiment of the blood pressure monitor each of the first and second fluid columns are in communication with at least one expansion bladder, which expansion bladder is configured to compensate for non-hydrostatic pressure components. To be able to determine a hydrostatic pressure offset that is solely a function of the height of the column of fluid, the fluid pressure in the fluid columns must be moderated for non-hydrostatic pressure components. Common non-hydrostatic pressure components include physical factors such as a compression of the fluid columns, which in a common instance are formed by flexible tubing, and environmental factors such as a temperature change of the fluid in the fluid columns. For this purpose, the expansion bladder is preferably formed by a fluid-containable reservoir, which reservoir is able to expand and contract upon changes in these physical and environmental factors. This expansion and contraction must be realised without exerting any pressure on the fluid due to the resilience of bladder itself. The reservoir may therefore be manufactured from a thin-walled, flexible and compliant material. On the outside, the reservoir may be surrounded by a fluid under a pressure equal to the preferred reference pressure prevalent in the fluid column at the location of the expansion bladder. The fluid surrounding the exterior of the reservoir thus provides a counter pressure equal to the pressure in the fluid column at the location of the expansion bladder. As the reference pressure is typically similar to the atmospheric pressure, the exterior of the reservoir may in a preferred instance be in direct contact with the outside air. The bladder may further comprise a casing for housing and thereby shielding the reservoir, to prevent the reservoir from getting disturbed externally. To retain direct contact with the outside air, the housing may be provided with one or more openings.

In a preferred embodiment, at least one expansion bladder is attached to the first end of each of the first and second fluid columns. Due to this position of the at least one expansion bladder, the pressure at the first end of the fluid columns corresponds to the reference pressure of the fluid surrounding the exterior of the bladder. As the one or more heart reference sensors are typically positioned at the second ends of the fluid columns, which ends are typically positioned below the first ends of the fluid columns, the placement of the at least one expansion bladder at the first ends of the fluid columns will yield positive relative pressures as measured by the one or more heart reference sensors. In a common instance, the first ends of the first and second fluid columns are each provided with an individual expansion bladder. The size of each of these bladders may differ and typically depend on the amount of fluid contained in the fluid column to which the respective bladder is connected. Specifically, the capacity of the bladder should preferably at least accommodate the range of pressure and volume changes experienced during normal operation of the blood pressure monitor and the heart reference unit in particular.

It is further preferred that each of the fluid columns and the at least one bladder in communication therewith form a gastight system that is closed off from its surrounding environment. To attain a gastight system, the at least one bladder and the fluid columns may be integrally connected to form a single part. Such an integrally formed part allows for a simple manufacturing process, yielding a more robust construction that can be produced in a cost-effective manner. The gas tightness prevents the heart reference unit from leaking and benefits the shelf life of the fluid contained in the fluid columns, as said fluid is not affected by the surrounding environment. With the right choice of fluid, the fluid could even have an virtually infinite shelf life. The gastight system further creates the possibility of choosing a reference pressure prevalent in the fluid columns that differs from the atmospheric pressure, wherein for instance a pressure above atmospheric pressure can be maintained in the fluid columns.

It is possible that the at least one heart reference sensor is configured for registering both positive and negative pressure differences between the first and second ends of the fluid columns. This allows the heart reference sensor to sense the pressure prevailing in the first fluid column and the second fluid column independent of relative vertical position of the ends of the fluid column, and independent of the placement of the heart reference sensor on the first or second end of the liquid columns. The fluid columns of the heart reference unit can hence be positioned in a way that is most convenient, without the necessity of placing the heart reference sensor at a lower end of the fluid columns. The heart reference sensor is preferably also configured for registering pressures both higher and lower than the pressure prevailing in the at least one expansion bladder, such that the position of the at least one bladder can be chosen freely.

In yet a further embodiment of the blood pressure monitor according to the invention, the fluid columns comprise a fluid-filled flexible tube. Due to their flexibility, the tubes are able to move with the patient and allow for the repositioning of both the first ends as well as the second ends of the fluid columns. In a typical instance, the fluid contained in each of the fluid columns has the same density. In this case, the measured hydrostatic pressure difference between the fluid columns is directly related to the elevation difference between the first end of the first and second fluid columns. The density of the fluid contained in the fluid columns may further be similar to, and preferably the same as the density of human blood. The measured hydrostatic pressure difference between the fluid columns is then similar to or the same as the offset between the measured blood pressure and the central arterial blood pressure, thus eliminating the need to correct the hydrostatic pressure difference for differences in densities between the fluids in the fluid columns and the patient's blood.

The invention further relates to a heart reference unit for a blood pressure monitor according to the invention, the benefits of which have been elaborated on with reference to the above-described blood pressure monitor.

The invention will now be elucidated into more detail with reference to a non-limitative exemplary embodiments shown in the following figures, wherein:
- figure 1 shows a perspective view of a blood pressure monitor according to the invention, and
- figure 2 shows a schematic view of another blood pressure monitor according to the invention.

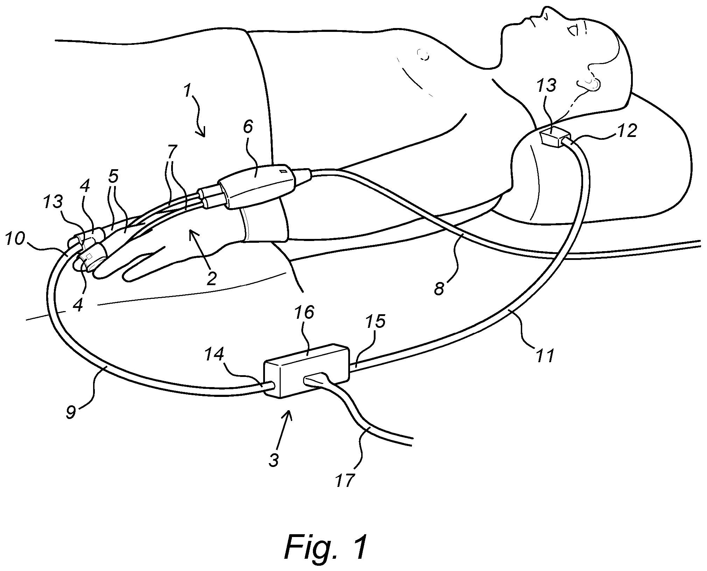

In figure 1, a blood pressure monitor (1) is shown, comprising a blood pressure sensor (2) and a heart reference unit (3). The blood pressure sensor (2) comprises two pressure cuffs (4), placed around a body extremity, in the depicted case a finger (5). The blood pressure sensor (2) further comprises a pressure controller (6) for controlling the pressure in the pressure cuffs (4), which pressure cuffs are connected to the pressure controller (6) by means of fluid lines (7). The pressure controller (6) is connectable to a power source through a power cable (8), which power cable may double as a data cable for the transfer of measurement data to a data processor and/or display device. The pressure cuffs (4) exert a pressure on the blood vessel walls in the body extremity, which pressure is controlled based on the signal of a plethysmograph build into the pressure cuffs (4). Although the blood pressure sensor (2) employs two pressure cuffs (4) that allow alternate measurements between two fingers (5), the use of a blood pressure sensor using a single cuff is likewise possible. The cuff pressure is controlled such that the signal of the plethysmograph, which is a measure of the volume of blood inside the blood vessels under the cuff, is kept constant. The counter pressure exerted by the pressure cuff (4) is then a direct measure for the actual blood pressure inside the blood vessel. This method is commonly applied for performing a continuous non-invasive blood pressure measurements, for which the blood pressure monitor (1) according to the invention is eminently suited. The invention however also includes blood pressure monitors utilizing different types of blood pressure sensors that for example use auscultatory or oscillometric methods for deriving blood pressures. Furthermore, the blood pressure sensor could be suited to be positioned at different locations on the body, such as a wrist or an upper arm, without deviating from the scope of the invention. The heart reference unit (3) comprises a first fluid column (9) with a first end (10) positioned at blood pressure sensor level and a second fluid column (11) with a first end (12) positioned at heart level. The fluid columns (9,11) are formed by flexible, fluid-filled tubes, which at their first ends (10,12) are connected to separate expansion bladders (13). The position of these expansion bladders (13) may however be chosen differently based on a preferred layout of the blood pressure monitor (1) and the heart reference unit (3) in particular. The second ends (14,15) of the fluid columns (9,11) opposing the first ends (10,12) of said fluid columns (9,11) are connected through a connector housing (16). This connector housing (16) houses a heart reference sensor (not shown) for sensing the hydrostatic pressure exerted by the first and second fluid column. The connector housing (16) further houses a differential pressure transducer (not shown) for determining a hydrostatic pressure difference between the first fluid column (9) and the second fluid column (11). The connector housing is connectable to a power source and/or a data processor by means of a (combined) power/data cable (17). The connector housing (16) may also comprise a wireless transmitter, for transmitted data and/or comprise its own power, for instance in the form of a battery.

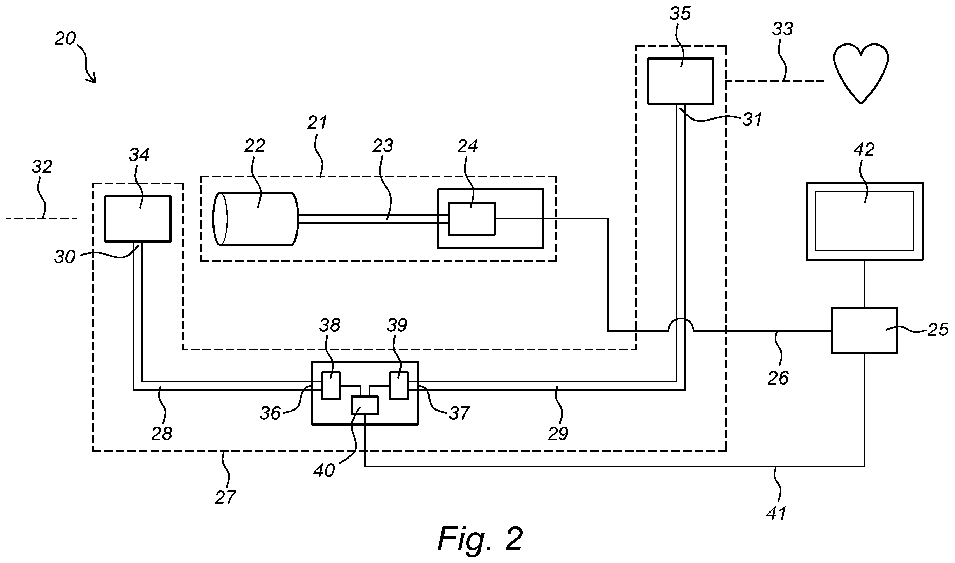

Figure 2 shows a schematic view of another blood pressure monitor (20) according to the invention. The blood pressure monitor (20) again comprises a blood pressure sensor (21) comprising a pressure cuff (22), via a fluid line (23) connected to a pressure controller (24). Pressure readings from the pressure controller (24) can be relayed to a processor (25) via a data cable (26). The blood pressure monitor further comprises a heart reference unit (27) comprising a first fluid column (28) and a second fluid column (29). The first end (30) of the first fluid column (28) and the first end (31) of the second fluid column (29) are respectively positioned at blood pressure measurement level (32) and at heart level (33). The fluid columns (28, 29) are at their first ends (30, 31) provided with a expansion bladder (34, 35). On the second ends (36,37) of the fluid columns, heart reference sensors (38,39) are provided for respectively sensing the hydrostatic pressure exerted by the first and second fluid columns (28,29). The heart reference sensors (38,39) are connected to a differential pressure transducer (40) for determining a hydrostatic pressure difference between the fluid columns (28,29). The differential pressure transducer (40) is further connected to the processor (25) via a data cable (41). The processor (25) is configured for deriving a central arterial pressure from the peripheral arterial pressure as measured by the blood pressure sensor (21) and the hydrostatic pressure difference determined by the differential pressure transducer (40). The determined central arterial pressure can subsequently be displayed by means of display means (42).

It will be apparent that the invention is not limited to the exemplary embodiments shown and described here, but that within the scope of the appended claims numerous variants are possible which will be self-evident to the skilled person in this field.

## Claims

1. A stand-alone heart reference unit (3; 27) for a blood pressure monitor (1; 20) that has a blood pressure sensor (2; 21) for non-invasive measurement of peripheral arterial blood pressure of a patient, the heart reference unit comprising
a first fluid column (9; 28) with positionable first and second ends (10; 30) and a second fluid column (11; 29) with positionable first and second ends (12; 31); wherein
- the first ends of said fluid columns are positionable near the patient at blood pressure sensor level and at a reference level; and
- the second ends of said fluid columns oppose the first ends (10,12; 30, 31) of said fluid columns (9, 11; 28, 29); the heart reference unit further comprising
a connector housing,
- which is positionable at a suitable location away from the blood pressure sensor and the patient's body and is connectable to a processor (25) via a wireless transmitter;
- to which the second ends of the first fluid column and the second fluid column are connectable so that the second ends have a predetermined relative elevation; and which
- in addition houses at least one heart reference sensor that includes a differential pressure transducer and is configured for registering and outputting both positive and negative hydrostatic pressure differences between the hydrostatic pressure exerted by the fluid in the first fluid column and the hydrostatic pressure exerted by the fluid in the second fluid column, measured by the at least one heart reference sensor, wherein the connector housing further houses electronics and electric wiring for connecting the at least one heart reference sensor such that the heart reference unit has all electronic components contained within the connector housing and no electronic connections outside the connector housing, the connector housing comprising a wireless transmitter for transmitted data and its own power.

2. The heart reference unit (3; 27) according to claim 1, comprising at least two heart reference sensors (38, 39), respectively positioned at the second end (14, 15; 36, 37) of the first (9; 28) and the second fluid column (11; 29).

3. The heart reference unit (3; 27) according to any of the preceding claims, wherein the at least one heart reference sensor (38, 39) is a bidirectional differential pressure sensor, for sensing a difference in pressure prevailing in the first fluid column (9; 28) and the second fluid column (11; 29).

4. The heart reference unit (3; 27) according to claim 3, wherein the second ends (14, 15; 36, 37) of the first fluid column (9; 28) and the second fluid column (11; 29) are mutually connected with the interposition of the differential pressure sensor.

5. The heart reference unit (3; 27) according to any of the preceding claims, wherein each of the first (9; 28) and second fluid columns (11; 29) are in communication with at least one expansion bladder (13; 34, 35), which expansion bladder (13; 34, 35) is configured to compensate for non-hydrostatic pressure components, wherein the at least one expansion bladder (13; 34, 35) is preferably attached to the first end (10, 12; 30, 31) of each of the first (9; 28) and second fluid columns (11; 29).

6. The heart reference unit (3; 27) according to claim 5, wherein each of the fluid columns (9, 11; 28, 29) and the at least one bladder (13; 34, 35) in communication therewith form a gastight system that is closed off from its surrounding environment.

7. The heart reference unit (3; 27) according to any of the preceding claims, wherein the fluid columns (9, 11; 28, 29) comprise a fluid-filled flexible tube.

8. The heart reference unit (3; 27) according to any of the preceding claims, wherein the fluid contained in each of the fluid columns (9, 11; 28, 29) has the same density, wherein the density of the fluid contained in the fluid columns (9, 11; 28, 29) is preferably similar to, and more preferably the same as the density of human blood.

9. A blood pressure monitor (1; 20) for blood pressure measurement, comprising:
a blood pressure sensor (2; 21) for measuring peripheral arterial pressure by a non-invasive blood pressure measurement at a location on the body, for instance at finger level; and
a heart reference unit (3; 27) according to any of the preceding claims.

10. The blood pressure monitor of claim 9, comprising a processor for deriving a central arterial pressure from the measured peripheral arterial pressure and the hydrostatic pressure difference.

## Patentansprüche

1. Eigenständige Herzreferenzeinheit (3; 27) für einen Blutdruckmonitor (1; 20), der einen Blutdrucksensor (2; 21) zur nicht-invasiven Messung des peripheren arteriellen Blutdrucks eines Patienten hat, wobei die Herzreferenzeinheit umfasst:
eine erste Fluidsäule (9; 28) mit positionierbaren ersten und zweiten Enden (10; 30), und eine zweite Fluidsäule (11; 29) mit positionierbaren ersten und zweiten Enden (12; 31); wobei
- die ersten Enden der Fluidsäulen nahe dem Patienten auf Blutdrucksensorniveau und auf einem Referenzniveau positionierbar sind; und
- die zweiten Enden der Fluidsäule den ersten Enden (10,12; 30, 31) der Fluidsäulen (9, 11; 28, 29) gegenüber liegen; wobei die Herzreferenzeinheit des Weiteren umfasst:
ein Verbindergehäuse,
- das an einer geeigneten Stelle von dem Blutdrucksensor und dem Patientenkörper entfernt positionierbar ist und mittels eines drahtlosen Senders mit einem Prozessor (25) verbindbar ist;
- mit dem die zweiten Enden der ersten Fluidsäule und der zweiten Fluidsäule verbindbar sind, so dass die zweiten Enden eine vorbestimmte relative Erhöhung aufweisen; und in dem
- zusätzlich mindestens ein Herzreferenzsensor untergebracht ist, der einen Differenzdruckwandler einschließt und ausgestaltet ist, um sowohl positive als auch negative hydrostatische Druckdifferenzen zwischen dem hydrostatischen Druck, der durch das Fluid in der ersten Fluidsäule ausgeübt wird, und dem hydrostatischen Druck, der durch das Fluid in der zweiten Fluidsäule ausgeübt wird, gemessen durch den mindestens einen Herzreferenzsensor, zu registrieren und auszugeben, wobei in dem Verbindergehäuse des Weiteren Elektronik und elektrische Verdrahtung zum Verbinden mit dem mindestens einen Herzreferenzsensor untergebracht sind, so dass die Herzreferenzeinheit alle elektronischen Komponenten aufweist, die innerhalb des Verbindergehäuses enthalten sind, und keine elektronischen Verbindungen außerhalb des Verbindergehäuses aufweist, wobei das Verbindergehäuse einen drahtlosen Sender zum Senden von Daten und seiner eigenen Leistung umfasst.

2. Herzreferenzeinheit (3; 27) nach Anspruch 1, umfassend mindestens zwei Herzreferenzsensoren (38, 39), die jeweils an dem zweiten Ende (14, 15; 36, 37) der ersten (9; 28) und der zweiten Fluidsäule (11; 29) positioniert sind.

3. Herzreferenzeinheit (3; 27) nach einem der vorhergehenden Ansprüche, wobei der mindestens eine Herzreferenzsensor (38, 39) ein bidirektionaler Differenzdrucksensor ist, um eine Differenz in dem in der ersten Fluidsäule (9; 28) und in der zweiten Fluidsäule (11; 29) vorherrschenden Druck abzufühlen.

4. Herzreferenzeinheit (3; 27) nach Anspruch 3, wobei die zweiten Enden (14, 15; 36, 37) der ersten Fluidsäule (9; 28) und der zweiten Fluidsäule (11; 29) wechselseitig mit der Zwischenposition des Differenzdrucksensors verbunden sind.

5. Herzreferenzeinheit (3; 27) nach einem der vorhergehenden Ansprüche, wobei jede der ersten (9; 28) und der zweiten Fluidsäule (11; 29) in Kommunikation mit mindestens einer Ausdehnungsblase (13; 34, 35) ist, wobei die Ausdehnungsblase (13; 34, 35) ausgestaltet ist, um nicht-hydrostatische Druckkomponenten zu kompensieren, wobei die mindestens eine Ausdehnungsblase (13; 34, 35) vorzugsweise an dem ersten Ende (10, 12; 30, 31) von jeder der ersten (9; 28) und zweiten Fluidsäulen (11; 29) angebracht ist.

6. Herzreferenzeinheit (3; 27) nach Anspruch 5, wobei jede der Fluidsäulen (9, 11; 28, 29) und der mindestens einen Blase (13; 34, 35) in Kommunikation damit ein gasdichtes System bildet, das von seiner umliegenden Umgebung abgeschlossen ist.

7. Herzreferenzeinheit (3; 27) nach einem der vorhergehenden Ansprüche, wobei die Fluidsäulen (9, 11; 28, 29) einen fluidgefüllten flexiblen Schlauch umfassen.

8. Herzreferenzeinheit (3; 27) nach einem der vorhergehenden Ansprüche, wobei das in jeder der Fluidsäulen (9, 11; 28, 29) enthaltene Fluid die gleiche Dichte hat, wobei die Dichte des in den Fluidsäulen (9, 11; 28, 29) enthaltenen Fluids vorzugsweise ähnlich der Dichte von menschlichem Blut und bevorzugter die gleiche Dichte wie diejenige von menschlichem Blut ist.

9. Blutdruckmonitor (1; 20) zur Blutdruckmessung, umfassend:
einen Blutdrucksensor (2; 21) zum Messen des peripheren arteriellen Drucks durch eine nicht-invasive Blutdruckmessung an einer Stelle am Körper, beispielsweise auf Fingerniveau; und
eine Herzreferenzeinheit (3; 27) nach einem der vorhergehenden Ansprüche.

10. Blutdruckmonitor nach Anspruch 9, umfassend einen Prozessor zum Ableiten eines zentralen arteriellen Drucks aus dem gemessenen peripheren arteriellen Druck und der hydrostatischen Druckdifferenz.

## Revendications

1. Unité de référence (3 ; 27) cardiaque autonome pour un dispositif de surveillance (1 ; 20) de tension artérielle qui présente un capteur (2 ; 21) de tension artérielle destiné à mesurer de manière non invasive la tension artérielle périphérique d'un patient, l'unité de référence cardiaque comprenant
une première colonne de fluide (9 ; 28) pourvue de première et seconde extrémités (10 ; 30) positionnables et une seconde colonne de fluide (11 ; 29) pourvue de première et seconde extrémités (12 ; 31) positionnables ;
- les premières extrémités desdites colonnes de fluide étant positionnables près du patient au niveau du capteur de tension artérielle et à un niveau de référence ; et
- les secondes extrémités desdites colonnes de fluide étant opposées aux premières extrémités (10, 12 ; 30, 31) desdites colonnes de fluide (9, 11 ; 28, 29) ; l'unité de référence cardiaque comprenant en outre
un boîtier de raccordement,
- qui peut être positionné en un emplacement approprié à distance du capteur de tension artérielle et du corps du patient et qui peut être connecté à un processeur (25) par l'intermédiaire d'un émetteur sans fil ;
- auquel les secondes extrémités de la première colonne de fluide et de la seconde colonne de fluide peuvent être raccordées de sorte que les secondes extrémités présentent une élévation relative prédéterminée ; et qui
- loge en outre au moins un capteur de référence cardiaque qui comprend un transducteur de pression différentielle et qui est conçu pour enregistrer et émettre à la fois des différences de pression hydrostatique positives et négatives entre la pression hydrostatique exercée par le fluide dans la première colonne de fluide et la pression hydrostatique exercée par le fluide dans la seconde colonne de fluide, mesurées par ledit au moins un capteur de référence cardiaque, le boîtier de raccordement logeant en outre une électronique et un câblage électrique destiné à connecter ledit au moins un capteur de référence cardiaque de telle sorte que l'unité de référence cardiaque présente tous les composants électroniques contenus à l'intérieur du boîtier de raccordement et aucune connexion électronique à l'extérieur du boîtier de raccordement, le boîtier de raccordement comprenant un émetteur sans fil pour des données transmises et sa propre alimentation.

2. Unité de référence (3 ; 27) cardiaque selon la revendication 1, comprenant au moins deux capteurs de référence cardiaque (38, 39), positionnés respectivement au niveau de la seconde extrémité (14, 15 ; 36, 37) de la première (9 ; 28) et de la seconde colonne de fluide (11 ; 29).

3. Unité de référence (3 ; 27) cardiaque selon l'une quelconque des revendications précédentes, ledit au moins un capteur de référence cardiaque (38, 39) étant un capteur de pression différentielle bidirectionnel, destiné à détecter une différence de pression régnant dans la première colonne de fluide (9 ; 28) et la seconde colonne de fluide (11 ; 29).

4. Unité de référence (3 ; 27) cardiaque selon la revendication 3, les secondes extrémités (14, 15 ; 36, 37) de la première colonne de fluide (9 ; 28) et de la seconde colonne de fluide (11 ; 29) étant mutuellement raccordées, le capteur de pression différentielle étant interposé.

5. Unité de référence (3 ; 27) cardiaque selon l'une quelconque des revendications précédentes, chaque colonne parmi les première (9 ; 28) et seconde colonnes de fluide (11 ; 29) étant en communication avec au moins une poche d'expansion (13 ; 34, 35), laquelle poche d'expansion (13 ; 34, 35) est conçue pour compenser des composants de pression non hydrostatique, ladite au moins une poche d'expansion (13 ; 34, 35) étant de préférence attachée à la première extrémité (10, 12 ; 30, 31) de chaque colonne parmi les première (9 ; 28) et seconde colonnes de fluide (11 ; 29).

6. Unité de référence (3 ; 27) cardiaque selon la revendication 5, chacune des colonnes de fluide (9, 11 ; 28, 29) et ladite au moins une poche (13 ; 34, 35) en communication avec celle-ci formant un système étanche aux gaz qui est fermé par rapport à son environnement ambiant.

7. Unité de référence (3 ; 27) cardiaque selon l'une quelconque des revendications précédentes, les colonnes de fluide (9, 11 ; 28, 29) comprenant un tube flexible rempli de fluide.

8. Unité de référence (3 ; 27) cardiaque selon l'une quelconque des revendications précédentes, le fluide contenu dans chacune des colonnes de fluide (9, 11 ; 28, 29) présentant la même masse volumique, la masse volumique du fluide contenu dans les colonnes de fluide (9, 11 ; 28, 29) étant de préférence similaire à la masse volumique du sang humain et plus préférablement identique à celle-ci.

9. Dispositif de surveillance (1 ; 20) de tension artérielle destiné à mesurer la tension artérielle, comprenant : un capteur (2 ; 21) de tension artérielle destiné à mesurer la tension artérielle périphérique par une mesure de tension artérielle non invasive en un emplacement sur le corps, par exemple au niveau du doigt ; et
une unité de référence (3 ; 27) cardiaque selon l'une quelconque des revendications précédentes.

10. Dispositif de surveillance de tension artérielle selon la revendication 9, comprenant un processeur destiné à déduire une tension artérielle centrale à partir de la tension artérielle périphérique mesurée et de la différence de pression hydrostatique.
